# EUROPEAN PATENT SPECIFICATION

(11) **EP 3 280 701 B1**
(45) Date of publication and mention of the grant of the patent: **16.10.2019**
(21) Application number: 16718846.5
(22) Date of filing: 11.04.2016
(51) Int. Cl.: C07D 209/48, C07C 315/06, C07C 317/28, C07C 235/16, C07C 315/04

(54) **A METHOD OF CHIRAL RESOLUTION OF THE KEY INTERMEDIATE OF THE SYNTHESIS OF APREMILAST AND ITS USE FOR THE PREPARATION OF PURE APREMILAST**
VERFAHREN ZUR CHIRALEN AUFLÖSUNG DES SCHLÜSSELINTERMEDIATS DER SYNTHESE VON APREMILAST UND DESSEN VERWENDUNG ZUR HERSTELLUNG VON REINEM APREMILAST
PROCÉDÉ DE RÉSOLUTION CHIRALE DE L'INTERMÉDIAIRE CLÉ DE LA SYNTHÈSE D'APRÉMILAST ET SON UTILISATION POUR LA PRÉPARATION D'APRÉMILAST PUR

(30) Priority: 09.04.2015 CZ 20150242
(43) Date of publication of application: 14.02.2018
(73) Proprietor: Zentiva K.S., 102 37 Praha 10 (CZ)
(72) Inventor: FAIGL, Ferenc, H-1114 Budapest (HU); DOUBSKY, Jan, 199 00 Praha 9 (CZ); KLVANA, Robert, 140 00 Praha 4 (CZ); RICHTER, Jindrich, 530 03 Pardubice (CZ); FOGASSY, Elemer, H-2030 Erd (HU); MATRAVOLGYI, Bela, H-1183 Budapest (HU); MIZSAK, Agnes, H-1181 Budapest (HU)
(74) Representative: Jirotkova, Ivana
(86) International application number: PCT/CZ2016/000038
(87) International publication number: WO 2016/161996

(56) References cited:
- EP-A1- 2 431 371
- WO-A1-03/080049
- WO-A1-2010/064212
- US-A- 4 034 113
- US-A1- 2013 217 919
- ELVERS B ET AL: "ULLMANN'S ENCYCLOPEDIA OF INDUSTRIAL CHEMISTRY, 3. OCCURRENCE AND PRODUCTION OF OPTICALLY ACTIVE COMPOUNDS", 1 January 1991 (1991-01-01), ULLMANN'S ENCYCLOPEDIA OF INDUSTRIAL CHEMISTRY, WEINHEIM. VCH.; DE, PAGE(S) 182 - 183, XP001167124, page 183
- MONTZKA T A ET AL: "SUBSTITUTED TARTRANILIC ACIDS. A NEW SERIES OF RESOLVING ACIDS", THE JOURNAL OF ORGANIC CHEMISTRY, AMERICAN CHEMICAL SOCIETY, US, vol. 33, no. 10, 1 October 1968 (1968-10-01), pages 3993-3995, XP002576424, ISSN: 0022-3263
- Robert T Standridge ET AL: "Phenylalkylamines with potential psychotherapeutic utility. 1. 2-Amino-1-(2,5-dimethoxy-4-methylphenyl)bu tane", Journal of Medicinal Chemistry, 1 December 1976 (1976-12-01), pages 1400-1404, XP055281727, DOI: 10.1021/jm00234a010 Retrieved from the Internet: URL:http://pubs.acs.org/doi/pdf/10.1021/jm 00234a010

## Description

### Technical Field

The invention relates to a process of preparing (*S*)-{2-[1-(3-ethoxy-4-methoxyphenyl)-2-methylsulfonylethyl]-4-acetylaminoisoindoline-1,3-dione of formula **1,** known as *Apremilast,* 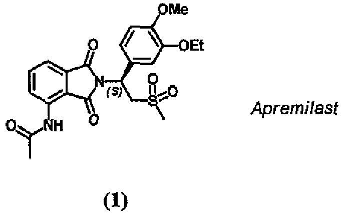 using chiral resolution of the key synthetic intermediate: racemic 1-(3-ethoxy-4-methoxyphenyl)-2-(methylsulfonyl)-ethylamine of formula **2a.** The racemic amine of formula **2a** is chirally resolved by means of derivatives of tartaric acid of formula **3,** and the resulting salts of formula **4a** can be used either directly (method *i, Scheme 1*), or after releasing of the free amine of formula (*S*)-2a by means of a suitable base (method ***ii**, Scheme 1*), for the preparation of pure *Apremilast* of formula **1** by subsequent reaction with 3-acetamidophtalic anhydride of formula **5.** In addition, method **ii** makes it possible to very easily and efficiently recover the resolving agent of formula **3.** 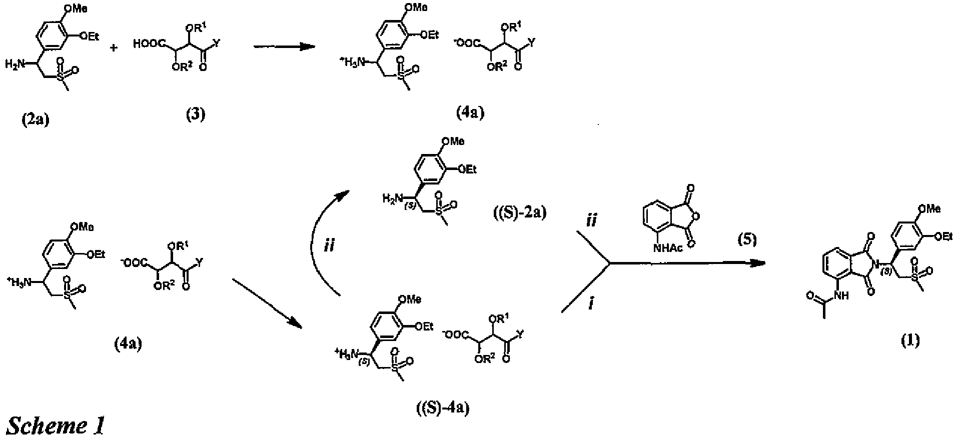 The key chiral intermediate of formula **(*S*)-2a** is isolated from the racemic amine of formula **2a** in the form of salts of formula **(*S*)-4a** with suitable derivatives of tartaric acid **3,** preferably with (*R,R*)-4-chlorotartranilic acid of formula **(*R,R*)-3a,** i.e. in the form of the salt of formula **(*S*)-4aa.**

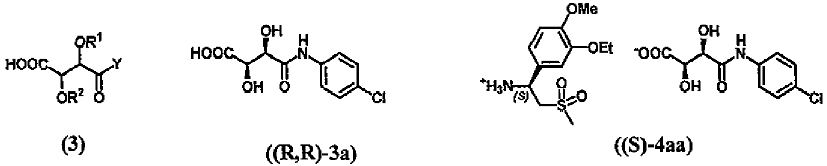

### Background Art

*Apremilast* of formula **1** is an orally administered *phosphodiesterase* 4 (PDE4) inhibitor, which inhibits spontaneous production of the *tumor necrosis factor α* (TNF-α), thus exhibiting an anti-inflammatory activity. It can be used for the treatment of psoriatic arthritis and it is also being tested for the treatment of other inflammatory diseases.

*Apremilast* of formula **1** was first described as a racemic mixture of pharmaceutically active ingredients (WO 2000/25777 A1; EP 1126839 B). A particular enantiomer, (*S*)-isomer, commonly only referred to as *Apremilast* of formula **1** was described in an application (WO 2003/080049) a few years later, which is the carrier of the proper biological activity. Thus, it is the chiral amine of formula **(*S*)-2a** that is the key intermediate for the synthesis of *Apremilast* of formula **1.** The application (WO 2003/080049) describes a procedure of chiral resolution of the racemic amine of formula **2a** by means of *N*-acetyl-L-leucine and subsequent use of the corresponding salt of formula **6** for the synthesis of *Apremilast* of formula **1** (*Scheme 2*)*.*

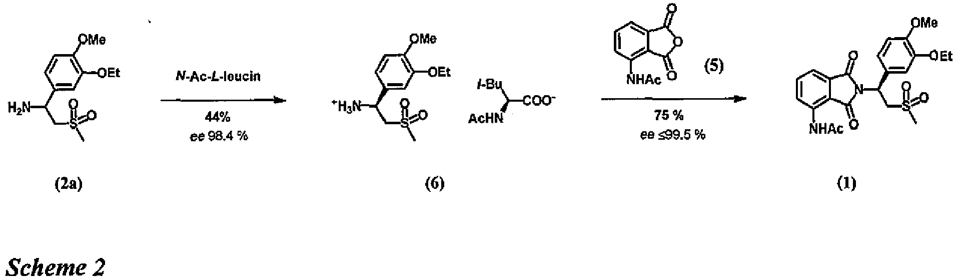

Using this procedure, the desired enantiomer of formula **(*S*)-2a** was isolated in the yield of 44%, or 89% (calculated on the theoretical content of 50% of formula **(*S*)-2a** in the racemic amine **2a**). Also, later applications (US 2008/0234359 A1; EP 2431371 A1) employ the use of *N*-acetyl-L-leucine, or of derivatives of chiral amino acids in general, for chiral resolution of the racemic amine of formula **2a** and the subsequent use of the corresponding salt of formula **6** for synthesis of *Apremilast* of formula **1** (*Scheme 2*)*.*

Besides the above mentioned ones, a number of asymmetrical syntheses of the desired enantiomer of formula **(*S*)-2a** have been described that use chiral catalysts of transition metals, e.g. rhodium (US2013/217919 A1; US2014/81032 A1). However, in economic terms, these procedures are not suitable for industrial production of (*S*)-1-(3-ethoxy-4-methoxyphenyl)-2-(methylsulfonyl)-ethylamine of formula **(*S*)-2a,** or *Apremilast* of formula **1.**

### Disclosure of Invention

The invention provides a process of preparing (*S*)-{2-[1-(3-ethoxy-4-methoxyphenyl)-2-methylsulfonylethyl]-4-acetylaminoisoindoline-1,3-dione of formula 1 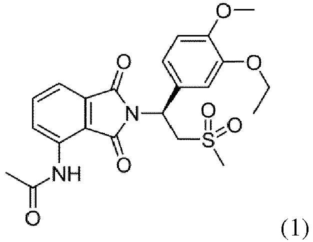 by means of a reaction of the racemic amine of formula 2a 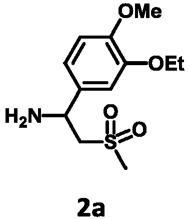 with derivatives of tartaric acid of formula **3,** namely with (*R,R*)-4-chlorotartranilic acid or (*R,R*)-di-*p*-toluoyl-tartaric acid, in a suitable solvent, preferably with the use of an additive, producing the salt of formula (*S*)-4a 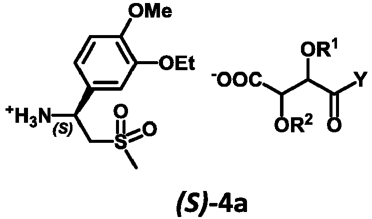 namely (*S*)-1-(3-ethoxy-4-methoxyphenyl)-2-(methylsulfonyl)-ethylamine (*R,R*)*-4-*chlorotartranilate, and (*S*)-1-(3-ethoxy-4-methoxyphenyl)-2-(methylsulfonyl)-ethylamine (*R,R*)-di-p-toluoyl-tartrate, resp.
and a subsequent reaction of the obtained salt of formula (*S*)-4a with 3-acetamidophtalic anhydride of formula 5, 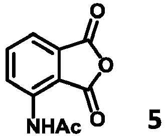 producing (*S*)-{2-[1-(3-ethoxy-4-methoxyphenyl)-2-methylsulfonylethyl]-4-acetylaminoisoindoline-1,3-dione of formula 1,
or conversion of the produced salt of formula (*S*)-4a in a suitable solvent with the use of a base to the free amine of formula (*S*)-2a 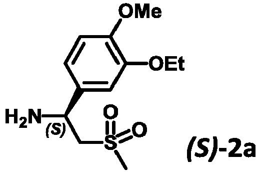 which, by a subsequent reaction with 3-acetamidophtalic anhydride of formula 5 provides (*S*)-{2-[1-(3-ethoxy-4-methoxyphenyl)-2-methylsulfonylethyl]-4-acetylaminoisoindoline-1,3-dione of formula 1.

The invention further provides *R,R*)-4-chlorotartranilate of (*S*)-1-(3-ethoxy-4-methoxyphenyl)-2-(methylsulfonyl)-ethylamine of formula (*S*)-4aa 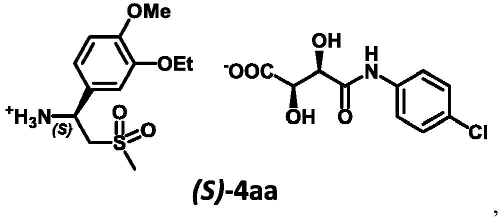 (*R,R*)-Di-p-toluoyl-tartrate of (*S*)-1-(3-ethoxy-4-methoxyphenyl)-2-(methylsulfonyl)-ethylamine of formula (*S*)-4ab 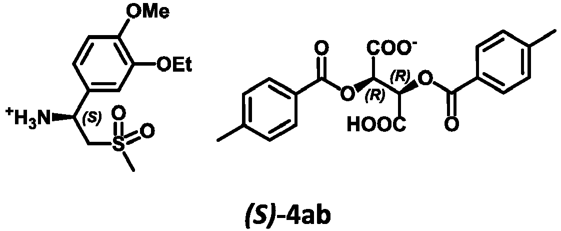 and the use of salts of formula **4a** in a process of preparing optically pure *Apremilast* of formula **1** (*Scheme 1*)*.*

It has been surprisingly found out that the derivatives of tartaric acid of formula **3** used, preferably (*R,R*)-4-chlorotartranilic acid of formula **(*R,R*)-3a,** make it possible to isolate (*S*)-1-(3-ethoxy-4-methoxyphenyl)-2-(methylsulfonyl)-ethylamine of formula **(*S*)-2a** in a high yield, with a high chemical and enantiomeric purity of above 99%. The described isolation methods can be easily transferred to the industrial scale to obtain a sufficient amount of the key chiral intermediate of formula **(*S*)-2a** for commercial use in the synthesis of *Apremilast* of formula **1.**

The derivatives of tartaric acid of formula **3** have the indisputable advantages of the possibility of using only about 0.5 molar equivalent of the chiral acid with respect to the amine of formula **2a,** of easy recycling for re-use, and also the possibility to carry out crystallization in water as the solvent. These all are significant innovative elements in terms of industrial production, its economy and environmental aspects.

Another clear advantage of the derivatives of tartaric acid of formula **3,** preferably (*R,R*)-4-chlorotartranilic acid of formula **(*R,R*)-3a,** is the fact that the salts of formula **(*S*)-4a,** preferably especially the salt of formula **(*S*)-4aa,** can be directly used in the preparation method of Apremilast of formula **1** without the necessity to release the corresponding amine of formula **(*S*)-2a** therefrom. From the point of view of industrial production and its economy, this fact also represents a substantial advantage.

### Detailed description of the invention

The invention provides a method of chiral resolution of racemic amines of the general structure of formula **2a** by means of derivatives of tartaric acid of formula **3** and the use thereof in the synthesis of *Apremilast* of formula **1.**

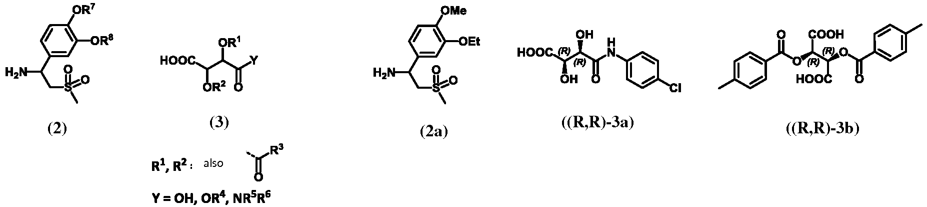

In the particular case of the racemic amine of formula 2a the derivatives of tartaric acid of formula (R,R)-3a and formula (R,R)-3b have proved to be especially suitable for chiral resolution. Both these acids are commercially available, namely also in the form of their optical antipodes of formula (S,S)-3a and formula (S,S)-3b and belong to ones of the cheapest resolution agents, which represents a significant advantage in terms of their prospective industrial use. (R,R)-4-Chlorotartranilic acid of formula (R,R)-3a can also be easily prepared even in kilogram amounts from the natural isomer of tartaric acid - (R,R), or L-(+)-tartaric acid - and 4-chloroaniline by processes described in literature (N. Huh, C. M. Thompson: Tetrahedron 1995, 51, 5935; T. Montzkat, E. Pindell, J. Matiskell: J. Org. Chem. 1968, 33, 3993).

The use of these acids makes it possible to isolate the respective salts of formula ***(S)-*4aa,** or formula **(*S*)-4ab,** in both high chemical (up to 95% with respect to the theoretical content of formula **(*S*)-2a** in the racemic amine of formula **2a**) and isomeric purities (*ee* >99%). The yield of the chiral resolution of the racemic amine of formula **2a** by means of these two acids, the chemical and isomeric purity of the obtained salts strongly depend on the reaction conditions. Above all on the solvent, temperature, reaction/crystallization time, stoichiometry and presence of other additives. The details are 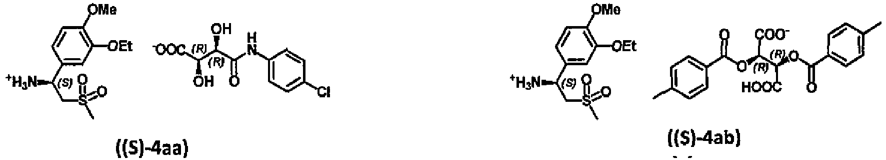 described below for the example of the salt of formula ***(S)*-4aa** and its preparation from the racemic amine of formula **2a** and (*R,R*)-4-chlorotartranilic acid of formula **(*R,R*)-3a.**

It has been surprisingly found out that, for very efficient chiral resolution of the racemic amine of formula **2a,** there is not needed a complete molar equivalent of (*R,R*)-4-chlorotartranilic acid of formula **(*R,R*)-3a** but that 0.4 to 1.0 molar equivalent of formula **(*R,R*)-3a** is a sufficient amount, both high yield and high isomeric purity being maintained. Preferably, as low as a 0.5 to 0.65 molar equivalent of formula **(*R,R*)-3a** can be used. The saving of ca. 1/2 of the chiral resolution agent represents a significant economic benefit for the resolution method of the racemic amine of formula **2a** in the industrial scale.

As further unexpectedly found out, the yield and isomeric purity of the salt of formula **(*S*)-4aa** can be also influenced by the presence of other additives having the character of strong acids. They are typically strong mineral acids, such as: sulfuric acid, phosphoric acid, perchloric acid and hydrochloric and hydrobromic acid. However, strong organic acids can be equally used, e.g. formic acid or sulfonic acids. In process, safety and economic terms, hydrochloric acid, or more precisely its aqueous solutions, have turned out to be the most suitable.

To achieve the highest possible chemical yields and isomeric purities of the desired salt **(*S*)-4aa,** the stoichiometry of all the components is of essential importance, i.e. of the racemic amine **2a,** (*R,R)*-4-chlorotartranilic acid of formula ***(R,R)*-3a** and hydrochloric acid **HCl.** As found out, the general molar ratio of formula **(*(R,R)*-3a** + **HCl)**:(amine of formula **2a**) should suitably be >1.0. Preferably, for this ratio to be 1:1, the amount of *(R,R)*-4-chlorotartranilic acid of formula ***(R,R)*-3a** varies in the range of 0.5-0.65 molar equivalents and the amount of hydrochloric acid **HCl** ranges between 0.45-0.6 molar equivalents.

The preparation of the crystalline salt of formula ***(S)*-4aa,** as well as its possible subsequent recrystallization to increase its isomeric purity, can be carried out in various solvents, the correct selection of which also influences the yield and isomeric purity of the desired product. From the economic, safety, process and environmental point of view - and even from the point of view of yields and isomeric purity - the most advantageous solvent is water itself.

However, various organic solvents or their mixtures with water can be equally advantageously used. Preferred organic solvents comprise: 1) C₁-C₆ aliphatic alcohols (preferably methanol and ethanol), 2) C₄-C₁₂ ethers (preferably cyclic ethers such as: tetrahydrofuran, 2-methyltetrahydrofuran nebo dioxane), 3) C₃-C₈ ketones (preferably acetone or ethyl methyl ketone), 4) C₃-C₁₂ esters (preferably ethyl acetate), 5) C₁-C₇ halogenated hydrocarbons (preferably dichloromethane, chloroform, saturated and unsaturated C₂-chloro derivatives, chlorobenzene, dichlorobenzenes or trifluoromethyl benzene), 6) C₅-C₁₂ hydrocarbons (preferably aromatic hydrocarbons such as toluene, or cyclic hydrocarbons such as cyclohexane and its derivatives) and finally 7) nitrogenous derivatives of hydrocarbons and carboxylic acids (preferably nitro derivatives and amides, or nitriles of C₁-C₆ carboxylic acids).

The actual preparation of the salt can be carried out in a wide temperature range. Beginning with low temperatures from -50°C (depending on the physical characteristics of the solvent used), across the room temperature up to the boiling point of the selected solvent or mixture of solvents (up to 150°C).

Although the formation of the salt of formula ***(S)*-4aa** is a very fast process under all the above mentioned conditions, to achieve an optimum yield and isomeric purity of formula ***(S)*-4aa** the reaction and crystallizing times are also important. As found out, the total reaction and crystallizing time should be suitably longer than 5 hours.

Concerning the method of chiral resolution of the racemic amine of formula **2a** by means of *(R,R)*-4-chlorotartranilic acid of formula ***(R,R)*-3a,** the process wherein 1 molar equivalent of the racemic amine of formula **2a** is reacted with 0.5-0.65 molar equivalent of *(R,R)*-4-chlorotartranilic acid of formula ***(R,R)*-3a** and 0.45-0.6 molar equivalent of hydrochloric acid **HCl** in water as the solvent, at temperatures between 10 and 80°C for 2-72 hours has proved as the most advantageous in every respect.

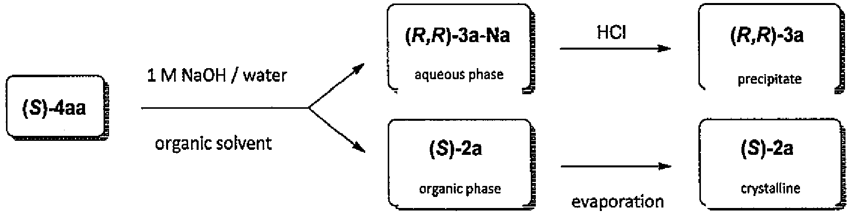

Besides the clear benefit that (*S*)-1-(3-ethoxy-4-methoxyphenyl)-2-(methylsulfonyl) ethylamine (*R,R*)-4-chlorotartranilate of formula **(*S*)-4aa** can be used directly for the synthesis of *Apremilast* of formula **1** (*Scheme 1, Scheme* 3), another substantial benefit of using this salt is the fact that it enables, in process terms, very easy recovery of the chiral resolution agent of formula ***(R,R)*-3a** after transformation of the salt of formula ***(S)*-4aa** to the respective free base of formula ***(S)*-2a.**

After releasing of the free amine of formula ***(S)*-2a** from the respective salt of formula ***(S)*-4aa,** one can use any water-immiscible and sufficiently strong base. Such bases are e.g. hydroxides, alkoxides, carbonates and hydrogen carbonates of alkali metals, soluble hydroxides and alkoxides of alkaline earth metals, or tetraalkylammonium hydroxides. Preferably, they are especially NaOH, LiOH, KOH, Na₂CO₃, NaHCO₃, K₂CO₃, KHCO₃ or Ba(OH)₂. The described method comprises mixing of the salt of formula ***(S)*-4aa** with one of the above mentioned bases in a mixture of water and in any water-immiscible solvent (*Figure* 6). The following compounds, e.g., can be used as suitable solvents: 1) C₄-C₁₂ ethers (preferably aliphatic ethers or cyclic ethers: diethyl ether, diisopropyl ether, *tert*-butyl methyl ether, 2-methyltetrahydrofuran and the like), 2) water-immiscible C₄-C₈ ketones (preferably ethyl methyl ketone), 3) C₃-C₁₂ esters (preferably C₁-C₆ alkyl acetates), 4) C₁-C₇ halogenated hydrocarbons (preferably dichloromethane, chloroform, saturated and unsaturated C₂-chloro derivatives, chlorobenzene, dichlorobenzenes or trifluoromethyl benzene), 5) C₅-C₁₂ hydrocarbons (preferably aromatic hydrocarbons such as toluene, or cyclic hydrocarbons such as cyclohexane and its derivatives) and finally 6) water-immiscible nitrogenous derivatives of hydrocarbons.

Another benefit of using *(R,R)*-4-chlorotartranilic acid of formula ***(R,R)*-3a** for chiral resolution of the racemic amine of formula 2a is also the fact that from the mother liquor after crystallization of the desired salt ***(S)*-4aa** the remaining fraction of the desired chiral amine of formula ***(S)*-2a** can be easily isolated in its racemic form of formula 2a while the opposite enantiomer of formula ***(R)*-2a** is obtained at the same time in a high isomeric purity, up to 97% *ee.*

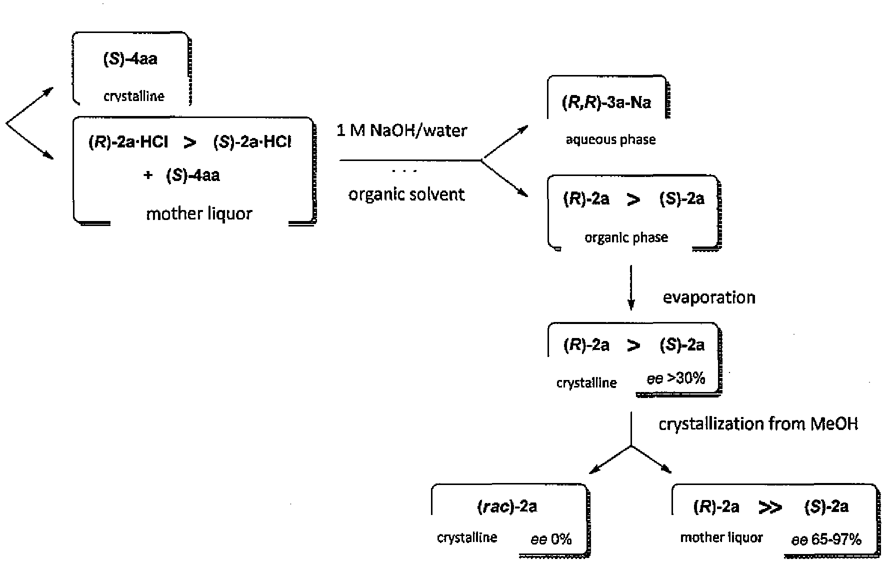

If the chiral resolution of the racemic amine of formula 2a is carried out under the conditions described above, the mother liquor can be, after the removing the desired salt of formula ***(S)*-4aa** by filtration, further processed and reused. An isomerically highly pure opposite isomer of formula ***(R)*-2a** can be obtained in this manner from the mother liquor, containing, besides a residual amount of the desired isomer of formula ***(S)*-2a,** the antipode of formula ***(R)*-2a** (both in the form of salts) in an isomeric purity of up to 97% *ee.* The base and water-immiscible organic solvents, used for the extraction of the mixture of free amines of formula ***(R)*-2a** and formula ***(S)*-2a,** are completely identical to the compounds mentioned above for releasing of the amine of formula ***(S)*-2a** from the salt of formula ***(S)*-4aa.** Crystallization of the isomeric mixture of the amines of formula **2a** with prevailing content of formula ***(R)*-2a** (*Figure 7*) can be conducted in various solvents, preferably in methanol, ethanol, acetone, ethyl acetate or tetrahydrofuran.

To synthesize *Apremilast* of formula 1, either directly (*R,R*)-4-chlorotartranilate of (*S*)-1-(3-ethoxy-4-methoxyphenyl)-2-(methylsulfonyl) ethylamine of formula ***(S)*-4aa,** or the corresponding free amine of formula ***(S)*-2a,** released from this salt under the conditions described above (*Scheme 3*)*,* can be used.

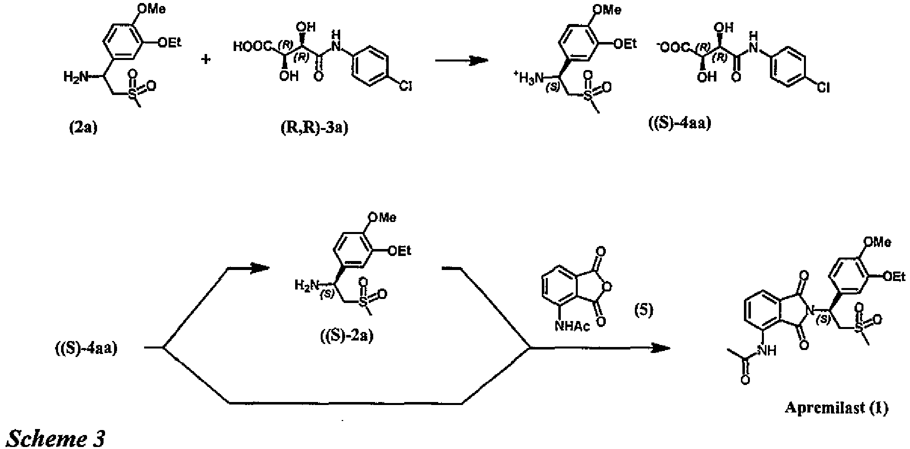

Both the have their advantages from the process and economic point of view. Direct use of the salt of formula ***(S)*-4aa** allows to reduce the number of process operations and - which is no less important - to isolate pure *Apremilast* 1 in a very high yield (85%), higher than in the case of the previously described salt of formula **6** (*Scheme 2*; yield 75%). If the free amine of formula ***(S)*-2a** is first isolated from the salt of formula ***(S)*-4aa** (using the above mentioned procedures) and is subsequently subjected to the reaction with the anhydride **5** (*Scheme 3*)*,* the number of process operations is higher, but on the other hand, the yield of the last reaction is higher (up to 89%) and in addition, the chiral resolution agent: (*R,R*)-4-chlorotartranilic acid of formula ***(R,R)*-3a** can be almost quantitatively recovered.

The condensation itself of the chiral amine of formula ***(S)*-2a,** or its salts of formula ***(S)*-4aa,** or formula ***(S)*-4ab** with the anhydride of formula **5** (*Scheme 3*) can be accomplished by their heating in a solution of a suitable organic solvent, in an aqueous solvent, or in mixtures of water with suitable organic solvents. To ensure trouble-free run of the reaction, both the reactants should be heated up to a temperature of >50°C, preferably to a temperature of 90-140°C. Especially C₂-C₅ carboxylic acids, preferably acetic acid, nitriles of C₂-C₅ carboxylic acids, preferably acetonitrile, and polar aprotic solvents such as dimethyl formamide, dimethyl acetamide, dimethyl sulfoxide, have proved to be suitable organic solvents. Besides them, hydrocarbons, aromatic hydrocarbons or their halogen derivatives can be used. Preferably, e.g,. toluene, xylenes, α,α,α-trifluorotoluene and *n*-butyl chloride.

The invention is clarified in a more detailed way using the examples below. The examples, which illustrate the improvement of the procedure in accordance with the invention, only have an illustrative character and do not restrict the scope of the invention in any respect.

### Experimental part

### High-performance liquid chromatography (HPLC)

The portion of the enantiomers of (*R*)- and (*S*)-1-(3-ethoxy-4-methoxyphenyl)-2-(methylsulfonyl)-ethylamine of formula **(*R*)-2a** and formula **(*S*)-2a** in their respective salts and mother liquors as well as the enantiomeric purity of all the intermediates and products were determined with the use of high-performance liquid chromatography (HPLC equipped with a UV/VIS detector). The analyses were conducted in a column with an AmyCoat® stationary phase (250x4.6 mm); 5 µm stationary phase) with the use of a hexane/2-propanol/diethylamine (65/35/1) mixture as the mobile phase (temperature 15°C; flow rate 0.8 ml/min; detection at 280 nm). Before the analysis, all the samples were dissolved in 2-propanol and diluted with hexane before the injection.

The term "room temperature" refers, for the purposes of the text below and above, to the temperature range from 22 to 26°C. Unless indicated otherwise, the term "equivalent" (or abbreviated "equiv.") always means "molar ratio" in the text and tables below. The indication *ee* means "enantiomeric excess" (in percent) of a pure isomer (*R* or *S*) in its mixture with the racemate (*RS*) mixture, and its calculation is based on the equation: *ee* = (|*R* - *S*|)/(*R* + *S*)·100 = [%*R* - %*S*][%].

### Examples

### Example 1

### Chiral resolution of the racemic amine of formula 2a with the use of various resolution agents (Table 1)

10 mg of the racemic amine of formula **2a** is weighed into a reaction vial, 100 µl of the respective (methanol, ethanol, acetone, acetonitrile, ethyl acetate, toluene, chloroform, tetrahydrofuran, 2-methyltetrahydrofuran, or 1,4-dioxane, or their mixtures with water) solvent is added and the mixture is moderately heated up until all the amine of formula **2a** is dissolved. Then, 0.5, or 1.0 molar equivalent of the respective resolution agent is added and the mixture is reheated to produce a clear solution. The reaction vial is closed and left to cool down to the room temperature. The formation of possible crystalline salts is monitored after 2, 4, 6, 24 and after 48 hours. Possible crystalline material and mother liquors are analyzed by means of HPLC.

**Table 1. (The various experiments are identified as: APR-number; no cryst. = no occurrence of any crystalline material was observed; the % figure refers to the chemical yield of the salt of formula 4a relative to the theoretical content of the enantiomer of formula (S)-2a; er = ratio of formula (S)/(R)-4a; the use of possible other solvents is specified directly for the particular experiments; successful experiments are identified by bold type.)**

| **Resolution agent** (name, *Mₘ*) | **MeOH** | | **Chloroform** | | **1,4-Dioxane** | | **Ethanol** | |
|---|---|---|---|---|---|---|---|---|
| *Equivalent of the resolution agent relative to* **2a:** | *0.5* | *1* | *0.5* | *1* | *0.5* | *1* | *0.5* | *1* |
| 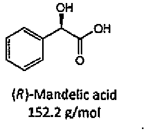 | APR-01 no cryst. | APR-01 no cryst. | APR-14 no cryst. | APR-14 no cryst. | APR-27 no cryst. | APR-27 no cryst. | - | - |
| 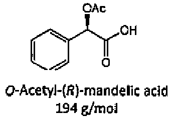 | APR-02 no cryst. | APR-02 no cryst. | APR-15 no cryst. | APR-15 no cryst. | APR-28 no cryst. | APR-28 no cryst. | - | - |
| 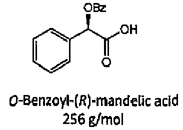 | APR-03 no cryst. | APR-03 no cryst. | APR-16 no cryst. | APR-16 no cryst. | APR-29 no cryst. | APR-29 no cryst. | - | - |
| 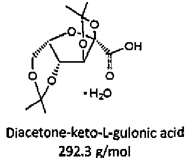 | APR-04 no cryst. | - | APR-17 no cryst. | - | APR-30 no cryst. | | - | - |
| 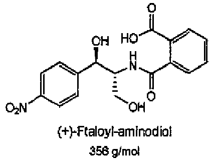 | APR-05 no cryst. | APR-05 no cryst. | APR-18 no cryst. | APR-18 no cryst. | APR-31 no cryst. | APR-31 no cryst. | - | - |
| 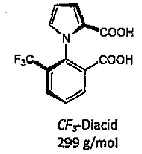 | APR-06 no cryst. | APR-06 no cryst. | APR-19 no cryst. | APR-19 no cryst. | APR-32 no cryst. | APR-32 no cryst. | - | - |
| 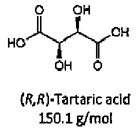 | APR-07 no cryst. | APR-07 no cryst. | APR-20 no cryst. | APR-20 no cryst. | APR-33 no cryst. | APR-33 no cryst. | - | - |
| 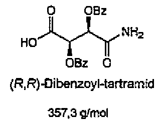 | APR-08 no cryst. | APR-08 no cryst. | APR-21 no cryst. | APR-21 no cryst. | APR-34 no cryst. | APR-34 no cryst. | - | - |
| 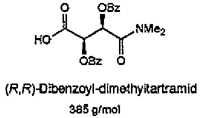 | APR-09 no cryst. | APR-09 no cryst. | APR-22 no cryst. | APR-22 no cryst. | APR-35 no cryst. | APR-35 no cryst. | - | - |
| 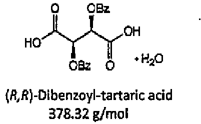 | APR-10 no cryst. | APR-10 no cryst. | APR-23 no cryst. | APR-23 no cryst. | APR-36 no cryst, | APR-36 no cryst. | - | - |
| 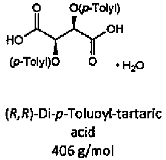 | APR-11 a small amount of crystals | **APR-11 117 %** *er*: **35/65** | APR-24 reagents insoluble | APR-24 reagents insoluble | **APR-37 58 % *er*: 38/62** | - | - | - |
| | ***0.5 equivalents of the resolution agent*** | | | | | | | |
| | solvents: EtOAc (APR-45), acetone (APR-46), MeCN (APR-49), EtOH (APR-50), toluene (APR-51) | | | | | | | |
| | *In all the cases the isolated salt **(S)-4a** also contained a considerable amount of the free amine **2a!*** | | | | | | | |
| 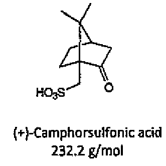 | APR-12 no cryst. | APR-12 no cryst. | APR-25 no cryst. | APR-25 no cryst. | APR-25 no cryst. | APR-39 129 % *er*: 50/50 | - | - |
| 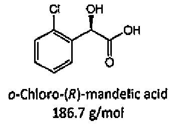 | APR-13 no cryst. | APR-13 no cryst. | APR-26 no cryst. | APR-26 no cryst. | APR-39 no Cryst. | APR-39 no cryst. | - | - |
| 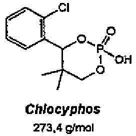 | APR-40 no cryst. | - | - | - | - | - | - | - |
| 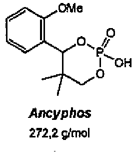 | APR-41 no cryst. | - | - | - | - | - | - | - |
| 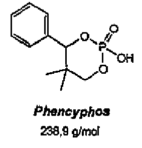 | APR-42 no cryst. | - | - | - | - | - | - | - |
| 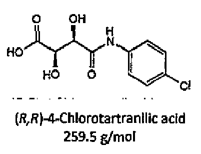 | APR-56 no cryst. | - | **APR-55 94 % *er*: 19/81** | - | **APR-57 104 % *er*: 12/88** | - | **APR-73 103 % *er*: 25/75** | - |
| 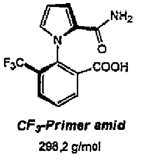 | **APR-44 94% *er*: 71/29** | - | **APR-43 113 % *er*: 83/17** | - | - | - | - | - |
| 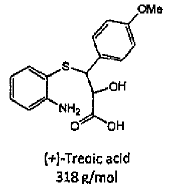 | APR-59 no cryst. | - | APR-58 no cryst. | - | APR-60 no cryst. | - | - | - |
| 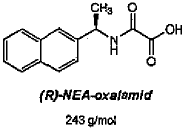 | APR-62 no cryst. | - | APR-61 no cryst. | - | APR-63 no cryst. | - | - | - |
| 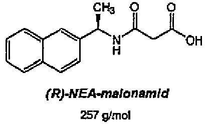 | APR-68 no cryst. | - | APR-67 no cryst. | - | APR-69 no cryst. | - | - | - |
| 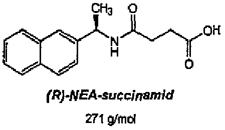 | APR-71 no cryst. | - | APR-70 no cryst. | - | APR-72 - no cryst. - | - | - | - |
| 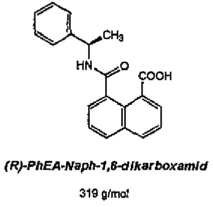 | APR-65 no cryst. | - | APR-64 no cryst. | - | APR-66 no cryst. | - | - | - |

### Example 2

### Chiral resolution of the racemic amine of formula 2a with the use of (R,R)-4-chlorotartranilic acid of formula (R,R)-3a under various conditions (Table 2)

10 mg of the racemic amine of formula **2a** is weighed into a reaction vial, 100 µl of the respective solvent is added, or possibly also the corresponding amount of hydrochloric acid, and the corresponding amount of (*R,R*)-4-chlorotartranilic acid of formula **(*R,R*)-3a.** A crystallizing seed of (*R,R*)-4-chloro-tartranilate of (*S*)-1-(3-ethoxy-4-methoxyphenyl)-2-(methylsulfonyl)-ethylamine of formula **(*S*)-4aa** is added to the clear solution produced after heating up, the vial is closed and left to cool down to the room temperature. The produced crystals are aspirated and analyzed directly by means of HPLC.

**Table 2 (The various experiments are identified as: APR-number; the yields refer to the isolated yields of the salt of formula (S)-4aa; er = ratio of formula (S)/(R)-4aa; the amount of the used solvent is specified as the volume multiple of the weight of the racemic amine of formula 2a - e.g. 10× means 1 g of formula 2a per 10 ml of the respective solvent; the best results are highlighted in red.)**

| | | | | | |
|---|---|---|---|---|---|
| | 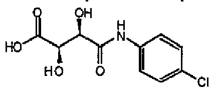 | | **(*R,R*)-3a** | | |
| | (*R,R*)-4--Chloroanthranilic acid 259.5 g/mol | | | | |

| **Solvent** | **Equivalent of *(R,R)*-3a** | **Experiment no.** | ***er*** | **Yield [%]** relative to the theoretical content of ***(S)*-2a** | **Yield [%]** relative to ***(R,R)*-3a** |
|---|---|---|---|---|---|
| **Dioxane** (wet - 0.48% H₂O) **10×** | **0.43** | **APR-75** | **1/99** | 73 | 77 |
| | 0.45 | APR-80 | 11/89 | 83 | 84 |
| | 0.47 | APR-81 | 17/83 | 79 | 77 |
| | 0.5 | APR-76 | 17/83 | - | |
| | 1 | APR-77 | 14/86 | - | |
| **Dioxane** (dry) **10×** | 0.43 | APR-83 | 21/79 | 74 | |
| **Dioxane** (wet - 1.0% H₂O) **10×** | 0.43 | APR-88 | 0.5/99.5 | 67 | |
| | 0.5 | APR-89 | 2/98 | 77 | |
| **Dioxane** (wet - 2.0% H₂O) **10×** | **0.5** | **APR-90** | **1/99** | **73** | |
| **Dioxane** (wet - 2.0% H₂O) 7× | 0.5 | APR-93 | 28/72 | 95 | |
| **Dioxane** (wet - 2.85% H₂O) 7x | 0.5 | APR-92 | 31/69 | 72 | |
| **Tetrahydrofuran** (wet - 2.0% H₂O) **10×** | **0.5** | **APR-94** | **2/98** | **69** | |
| 2-**Methyltetrahydro furan** (wet - 2.0% H₂O) **10×** | 0.5 | APR-95 | 17/83 | 87 | |
| **Methanol** - **H₂O 9:1 10×** | 0.5 | APR-86 | 42/58 | 73 | |
| **H₂O 10×** | 0.5 | APR-82 | 29/71 | 112 | |
| **H₂O 10×** | 0.43 | APR-84 | 35/65 | 67 | |
| **H₂O** + **0.5 equiv. HCl 10×** | 0.5 | APR-87 | 7/93 | 67 | *without a crystallizing seed* |
| **H₂O** + **0.5 equiv. HCl 10×** | 0.5 | APR-96 | 2.5/97.5 | 67 | |
| **H₂O** + **0.53 equiv. HCl 10×** | 0.5 | **APR-98** | **2/98** | **69** | |
| **H₂O + 0.55 equiv. HCl 10×** | 0.5 | APR-91 | 2/98 | 64 | |

| In a larger scale 3.0 g 2a / water (mechanical stirrer) | | | | | |
|---|---|---|---|---|---|
| **H₂O + 0.53 equiv. HCl 10×** | **0.5** | **APR-100** | **0.5/99.5** | 77 | |

### Example 3

### (R,R)-4-chlorotartranilate of (S)-1-(3-ethoxy-4-methoxyphenyl)-2-(methylsulfonyl)-ethylamine of formula (S)-4aa

The racemic amine **2a** (3.00 g; 10.97 mmol; 1 equiv.) is dissolved in a mixture of 24.2 ml of water and 5.82 ml of a 1M aqueous solution of hydrochloric acid (0.53 equiv.) at 60-70°C in a flask equipped with a mechanical stirrer. Then, under continuous stirring, the amount of 1.43 g (5.485 mmol; 0.5 equiv.) of (*R,R*)-4-chlorotartranilic acid of formula **(*R,R*)-3a** is added and heating continues for another 10-15 min. A crystallizing seed of the salt of formula **(*S*)-4aa** is added to the resulting clear solution at the same temperature. The mixture is left to slowly cool down to the room temperature and the stirring continues overnight. The resulting crystals are aspirated, washed with water (2 x 2 ml) and dried at the room temperature. This provides 2.25 g of the product of formula **(*S*)-4aa** (yield 77%), *ee* 99.0% (HPLC), melting point 186°C (DSC).
¹H-NMR (DMSO-*d*₆): 1.33 (t, 3H), *J*=6.95 Hz; 2.90 (s, 3H); 3.42-3.56 (m, 2H); 3.74 (s, 3H); 3.99-4.07 (m, 2H); 4.27 (d, 1H), *J*=2.11; 4.36 (d, 1H), *J*=2.15; 4.37-4.42 (m, 1H); 6.89-6.97 (m, 2H); 7.09 (d, 1H), *J*=1.35; 7.35 (d, 2H), *J*=8.91; 7.77 (d, 2H), *J*=8.90; 9.76 (s, 1H). ¹³C-NMR (DMSO-*d*₆) δ: 11.78; 41.76; 50.49; 55.52; 60.48; 63.69; 72.04; 73.55; 111.67; 111.69; 119.05; 121.02; 126.94; 128.46; 134.47; 137.54; 147.97; 148.51; 171.21; 173.71.

### Example 4

### (R,R)-4-chlorotartranilate of (S)-1-(3-ethoxy-4-methoxyphenyl)-2-(methylsulfonyl)-ethylamine of formula (S)-4aa

The racemic amine **2a** (1.50 g; 5.49 mmol; 1 equiv.) is dissolved in a mixture of 12.53 ml of water and 2.47 ml of a 1M aqueous solution of hydrochloric acid (0.45 equiv.) at 60-70°C in a flask equipped with a mechanical stirrer. Then, under continuous stirring, the amount of 0.926 g (3.134 mmol; 0.65 equiv.) of (*R,R*)-4-chlorotartranilic acid of formula **(*R,R*)-3a** is added and heating continues for another 10-15 min. A crystallizing seed of the salt of formula **(*S*)-4aa** is added to the resulting clear solution at the same temperature. The mixture is left to slowly cool down to the room temperature and the stirring continues overnight. The resulting crystals are aspirated, washed with water (3 x 3 ml) and dried at the temperature of 46-50 °C. This provides 1.34 g of the product of formula **(*S*)-4aa** (yield 92%), *ee* >99% (HPLC), melting point 186°C (DSC).

### Example 5

### Recrystallization of (R,R)-4-chlorotartranilate of (S)-1-(3-ethoxy-4-methoxyphenyl)-2-(methylsulfonyl)-ethyl-amine of formula (S)-4aa from water (initial ee 92%)

The diastereoisomeric salt of formula **(*S*)-4aa** (3.50 g; *ee* 92%) is dissolved in 35 ml of distilled water in a hot state and then, under stirring, it is left to cool down to the room temperature. At this temperature it is stirred for another 2 hours. The resulting crystals are aspirated, washed with water (3 x 3 ml) and dried at 46-50°C. This provides 2.98 g (yield 85%) of the product of formula **(*S*)-4aa** at *ee* >99%.

### Example 6

### Recrystallization of (R,R)-4-chlorotartranilate of (S)-1-(3-ethoxy-4-methoxyphenyl)-2-(methylsulfonyl)-ethyl-amine of formula (S)-4aa from water (initial ee 96.6%)

The diastereoisomeric salt of formula **(*S*)-4aa** (1.265 g; *ee* 96.6%) is dissolved in 10.2 ml of distilled water in a hot state and then, under stirring, it is left to cool down to the room temperature. At the room temperature it is stirred for another 2 hours. The resulting crystals are aspirated, washed with water (3 x 1 ml) and dried at 46-50°C. This provides 1.066 g (yield 84%) of the product of formula **(*S*)-4aa** at *ee* >99%.

### Example 7

### Recrystallization of (R,R)-4-chlorotartranilate of (S)-1-(3-ethoxy-4-methoxyphenyl)-2-(methylsulfonyl)-ethyl-amine of formula (S)-4aa from methanol (initial ee 95%)

The diastereoisomeric salt of formula **(*S*)-4aa** (4.00 g; *ee* 95%) is dissolved in 220 ml of methanol in a hot state and then, under stirring, it is left to cool down to the room temperature. At the room temperature it is stirred for another 2 hours. The resulting crystals are aspirated, washed with methanol (3 x 10 ml) and dried at 46-50°C. This provides 2.84 g (yield 71%) of the product of formula **(*S*)-4aa** at *ee* 99.9% and the chemical purity of 99.97% (HPLC).

### Example 8

### (R,R)-Di-p-toluoyl-tartrate of (S)-1-(3-ethoxy-4-methoxyphenyl)-2-(methylsulfonyl)-ethylamine of formula (S)-4ab

The racemic amine **2a** (3.00 g; 10.97 mmol) is dissolved in 26 ml of MeOH at 50-65°C in a flask fitted with a mechanical stirrer. Then, under continuous stirring, the amount of 4.44 g (10.97 mmol) of (*R,R*)-di-*p*-toluoyl-tartaric acid monohydrate of formula **(*R,R*)-3b** is added and heating continues for another 10-15 min. A crystallizing seed of the salt of formula **(*S*)-4ab** is added to the resulting clear solution at the same temperature. The mixture is left to slowly cool down to the room temperature and the stirring continues overnight. The resulting crystals are aspirated, washed with icy methanol (2 x 2 ml) and dried at the room temperature. This provides 4.27 g of the product of formula **(*S*)-4ab** (yield 59%), *ee* 30.0% (HPLC).

### Example 9

### Isolation of free (S)-1-(3-ethoxy-4-methoxyphenyl)-2-(methylsulfonyl)-ethylamine of formula (S)-2a

The diastereoisomeric salt of formula **(*S*)-4aa** from *Example 5* (1.27 g; 2.38 mmol; *ee* >99%) is stirred up in a mixture of a 1M aqueous solution of NaOH (8.70 ml; 8.7 mmol) and 20 ml of dichloromethane at the room temperature. After 20 minutes the stirring is stopped, the organic phase is separated and the aqueous phase is extracted with 2 x 20 ml of dichloromethane. The joined organic phases are dried with anhydrous Na₂SO₄ and subsequently evaporated on an evaporator at a reduced pressure. This provides 0.61 g (yield 94%) of the pure amine of formula **(*S*)-2a** as a white solid substance (ee 99.4%).

### Example 10

### Isolation of free (S)-1-(3-ethoxy-4-methoxyphenyl)-2-(methylsulfonyl)-ethylamine of formula (S)-2a

The diastereoisomeric salt of formula **(*S*)-4aa** from *Example 5* (1.27 g; 2.38 mmol; *ee* >99%) is stirred up in a mixture of 10 ml of an aqueous solution of Na₂CO₃ (750 mg; 7.08 mmol) and 20 ml of ethyl acetate at 35°C. After 30 minutes the stirring is stopped, the organic phase is separated and the aqueous phase is extracted with 2 x 20 ml of ethyl acetate. The joined organic phases are dried with anhydrous Na₂SO₄ and subsequently evaporated on an evaporator at a reduced pressure. This provides 0.58 g (yield 89 %) of the pure amine of formula **(*S*)-2a** as a white solid substance (ee 99.3%).

### Example 11

### Regeneration of (R,R)-4-chlorotartranilic acid of formula (R,R)-3a

The aqueous phase from *Example 9* is acidified with concentrated aqueous HCl (approx. 1.0 ml) to pH = 1 while being stirred, the separated precipitate is aspirated and washed with 3 x 1 ml of water. After air-drying the amount of 0.47 g (yield 75%) of the pure acid of formula **(*R,R*)-3a** is obtained (melting point 191-193°C; [α]_{D}: + 106.2 (c 1, MeOH)).

### Example 12

### Isolation of (rac)-1-(3-ethoxy-4-methoxyphenyl)-2-(methylsulfonyl)ethylamine of formula 2a and (R)-1-(3-ethoxy-4-methoxyphenyl)-2-(methylsulfonyl)ethylamine of formula (R)-2a from the mother liquors after crystallization of the compound of formula (S)-4aa

Pure salt of formula **(*S*)-4aa** is obtained from 3.0 g (10.98 mmol) of the racemic amine of formula **2a** through the method described in *Example 4.* The amount of 0.93 g of NaOH (23.3 mmol) is added to the aqueous mother liquors and the released amine (mixture of **(*R*)-2a** > **(*S*)-2a)** is extracted with dichloromethane (3x 20 ml). The organic phase is dried with anhydrous Na₂SO₄ and subsequently evaporated on an evaporator at a reduced pressure. This provides 1.50 g of a mixture of **(*R*)-2a** > **(*S*)-2a** (*ee* 54%). The white solid substance obtained this way is dissolved in 12 ml of methanol in a hot state, inoculated with the racemic amine of formula **2a** and stirred overnight at the room temperature. After aspiration of the produced crystals and washing with methanol (2x 1 ml) the amount of 0.48 g (yield 70%) of the racemic amine of formula **2a** (*ee* 0%) is obtained. Evaporation of the mother liquors can provide the compound of formula **(*R*)-2a** (1.01 g; *ee* 79%)

### Example 13

### (S)-{2-[1-(3-ethoxy-4-methoxyphenyl)-2-methylsulfonylethyl]-4-acetylaminoisoindoline-1,3-dione of formula 1

14.92 g (28 mmol; >99 % *ee)* of (*S*)-1-(3-ethoxy-4-methoxyphenyl)-2-(methylsulfonyl)-ethylammonium-(*R,R*)-4-chlorotartanilate of formula **(*S*)-4aa,** 6.05 g (29.4 mmol) of 3-acetamidophtalic anhydride of formula **5** and 125 ml of glacial acetic acid were placed into a 250 ml flask. The mixture was refluxed overnight and then cooled down to <50°C. Then, the solution was concentrated in vacuum and the residue was dissolved in 400 ml of ethyl acetate. The obtained solution was washed with water (2 x 125 ml), saturated aqueous solution of NaHCO₃ (2 x 125 ml), salt brine (2 x 125 ml) and dried with sodium sulphate. The solvent was evaporated at a reduced pressure and the rest was then crystallized from a mixture of 75 ml of ethanol and 37.5 ml of acetone. The separated crystals were aspirated on frit and washed with ethanol (2 x 50 ml). The product was dried at a reduced pressure (0.5 kPa) at 60°C until a constant weight was achieved, providing 9.55 g of the product of formula 1 (yield 74%, >99 % *ee,* HPLC 99.2%).
¹H-NMR (CDCl₃) δ: 1.46 (t, 3H); 2.26 (s, 3H); 2.87 (s, 3H); 3.65-3.78 (dd, 1H); 3.84 (s, 3H); 4.10 (q, 2H); 4.48-4.62 (dd, 1H); 5.84-5.90 (dd, 1H); 6.79-6.89 (d, 1H); 7.03-7.15 (m, 2H); 7.44-7.52 (d, 1H); 7.59-7.71 (m, 1H); 8.70-8.80 (d, 1H); 9.45 (s, 1H). ¹³C-NMR (CDCl₃) δ: 14.69; 24.96; 41.64; 48.57; 54.51; 55.95; 64.54; 111.47; 112.42; 115.13; 118.24; 120.31; 124.98; 129.26; 131.06; 136.13; 137.64; 148.66; 149.78; 167.50; 169.16; 169.52.

### Example 14

### (S)-{2-[1-(3-ethoxy-4-methoxyphenyl)-2-methylsulfonylethyl]-4-acetylaminoisoindoline-1,3-dione of formula 1

14.92 g (28 mmol; >99 % *ee)* of (*S*)-1-(3-ethoxy-4-methoxyphenyl)-2-(methylsulfonyl)-ethylammonium-(*R,R*)-4-chlorotartanilate of formula **(*S*)-4aa,** 6.05 g (29.4 mmol) of 3-acetamidophtalic anhydride of formula **5** and 125 ml of glacial acetic acid were placed into a 250 ml flask. The mixture was refluxed for 24 h and then cooled down to <50°C. Then, the solution was concentrated in vacuum and the residue was dissolved in 400 ml of ethyl acetate. The obtained solution was washed with water (2 x 125 ml), saturated aqueous solution of NaHCO₃ (2 x 125 ml), salt brine (2 x 125 ml) and dried with sodium sulphate. The solvent was evaporated at a reduced pressure and the rest was then crystallized from a mixture of ethanol - acetone 2:1 (by volume) The separated crystals were aspirated on frit and washed with ethanol (2 x 50 ml). The product was dried at a reduced pressure (0.5 kPa) at 60 °C until a constant weight was achieved, providing 11.02 g of the product of formula **1** (yield 85%, *>99% ee,* HPLC 99.7%).

### Example 15

### (S)-{2-[1-(3-ethoxy-4-methoxyphenyl)-2-methylsulfonylethyl]-4-acetylaminoisoindoline-1,3-dione of formula 1

7.65 g (28 mmol; 99.4% *ee)* of (*S*)-1-(3-ethoxy-4-methoxyphenyl)-2-(methylsulfonyl)-ethylamine of formula **(*S*)-2a** (see *Example 9*), 6.05 g (29.4 mmol) of 3-acetamidophtalic anhydride of formula 5 and 125 ml of glacial acetic acid were placed into a 250ml flask. The mixture was refluxed overnight and then cooled down to <50°C. Then, the solution was concentrated at a reduced pressure and the residue was dissolved in 400 ml of ethyl acetate. The obtained solution was washed with water (2 x 125 ml), saturated aqueous solution of NaHCO₃ (2 x 125 ml), salt brine (2 x 125 ml) and dried with sodium sulphate. The solvent was evaporated at a reduced pressure and the rest was crystallized from a mixture of 75 ml of ethanol and 37.5 ml of acetone. The separated crystals were isolated by filtration on frit and washed with ethanol (2 x 50 ml). The product was dried at a reduced pressure (0.5 kPa) at 60°C until a constant weight was achieved, providing 9.56 g of the product of formula **1** (yield 74 %, >99% *ee*, HPLC 99.4%).

### Example 16

### (S)-{2-[1-(3-ethoxy-4-methoxyphenyl)-2-methylsulfonylethyl]-4-acetylaminoisoindoline-1,3-dione of formula 1

3.17 g (11,6 mmol; 99.4 % *ee)* of (*S*)-1-(3-ethoxy-4-methoxyphenyl)-2-(methylsulfonyl)-ethylamine of formula **(*S*)-2a** (see *Example 9*), 2.50 g (12.2 mmol) of 3-acetamidophtalic anhydride of formula **5** and 52 ml of glacial acetic acid were placed into a 250 ml flask. The mixture was refluxed for 3 h and cooled down to 20°C. After that, 4 x 25 ml of water were gradually added, under continuous stirring the mixture was inoculated with crystals of *Apremilast* of formula **1** and stirred at 20 °C. The separated crystals were aspirated, washed with a mixture of acetic acid - water (volume ratio 2:5) and dried at a reduced pressure. The amount of 4.75 g of yellowish crystals of the product of formula **1** was obtained (yield 89%, >99% *ee*, HPLC 99.1%).

### Example 17

### (S)-{2-[1-(3-ethoxy-4-methoxyphenyl)-2-methylsulfonylethyl]-4-acetylaminoisoindoline-1,3-dione of formula 1

*Apremilast* of formula **1** was prepared from the salt of formula **(*S*)-4ab** *(Example 8)* and 3-acetamidophtalic anhydride of formula **5** according to the procedure described in *Example 14.*

4.12 g (6,25 mmol; 30 % *ee)* of (*R,R*)-di-*p*-toluoyl-tartrate of (*S*)-1-(3-ethoxy-4-methoxyphenyl)-2-(methylsulfonyl)-ethylamine of formula **(*S*)-4ab,** 1.35 g (6.56 mmol) of 3-acetamidophtalic anhydride of formula 5 and 25 ml of glacial acetic acid were placed into a 50 ml flask. The mixture was refluxed for 24 h and then cooled down to 40°C. Then, the solution was concentrated in vacuum and the residue was dissolved in 100 ml of ethyl acetate. The obtained solution was washed with water (2 x 30 ml), saturated aqueous solution of NaHCO₃ (2 x 30 ml), salt brine (2 x 30 ml) and dried with sodium sulphate. The solvent was evaporated at a reduced pressure and the rest was then crystallized from a mixture of ethanol - acetone 2:1 (by volume) The separated crystals were aspirated on frit and washed with ethanol (2 x 10 ml). The product was dried at a reduced pressure (0.5 kPa) at 60°C until a constant weight was achieved. This way, 2.39 g of the product of formula 1 was obtained (yield 83%; *ee* 30%; HPLC 99.7%).

## Claims

1. A process of preparing (*S*)-{2-[1-(3-ethoxy-4-methoxyphenyl)-2-methylsulfonylethyl]-4-acetylaminoisoindoline-1,3-dione of formula 1 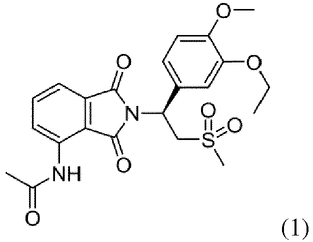 **characterized in that** it comprises a reaction of the racemic amine of formula 2a 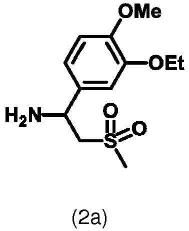 with (*R,R*)-4-chlorotartranilic acid or (*R,R*)-di-*p*-toluoyl-tartaric acid in a suitable solvent, preferably with the use of an additive, producing the salt of formula (*S*)-4a 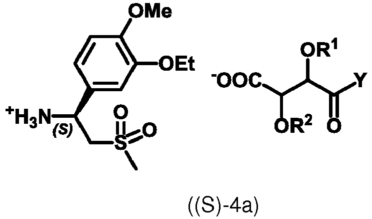 namely (*S*)-1-(3-ethoxy-4-methoxyphenyl)-2-(methylsulfonyl)-ethylamine (*R,R*)-4-chlorotartranilate, and (*S*)-1-(3-ethoxy-4-methoxyphenyl)-2-(methylsulfonyl)-ethylamine (*R,R*)-di-p-toluoyl-tartrate, resp.,
and a subsequent reaction of the obtained salt of formula (*S*)-4a with 3-acetamidophtalic anhydride of formula 5, 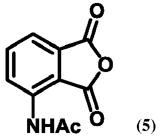 producing (*S*)-{2-[1-(3-ethoxy-4-methoxyphenyl)-2-methylsulfonylethyl]-4-acetylaminoisoindoline-1,3-dione of formula 1,
or conversion of the produced salt of formula (*S*)-4a in a suitable solvent with the use of a base to the free amine of formula (*S*)-2a, 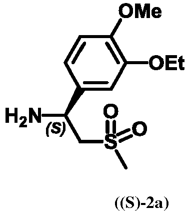 which, by a subsequent reaction with 3-acetamidophtalic anhydride of formula 5 provides (*S*)-{2-[1-(3-ethoxy-4-methoxyphenyl)-2-methylsulfonylethyl]-4-acetylaminoisoindoline-1,3-dione of formula 1.

2. The process according to claim 1, **characterized in that** the molar ratio of the racemic amine and the derivative of tartaric acid is 1:0.5 to 1:0.65.

3. The process according to claims 1 to 2, **characterized in that** said additive is selected from the group consisting of mineral acids comprising sulfuric, phosphoric, perchloric, hydrochloric and hydrobromic acid, and organic acids comprising formic acid or sulfonic acids, preferably hydrochloric acid.

4. The process according to claim 3, **characterized in that** the molar ratio of the racemic amine, derivative of tartaric acid and additive is 1: 0.50 to 0.65 : 0.45 to 0.60, preferably 1:0.65:0.45.

5. The process according to any one of the preceding claims, **characterized in that** the solvent for the reaction with the derivatives of tartaric acid is selected from the group consisting of water, C1-C6 aliphatic alcohols, C4-C12 ethers, C3-C8 ketones, C3-C12 esters, C1-C7 halogenated hydrocarbons, C5-C12 hydrocarbons, nitrogen derivatives of hydrocarbons and carboxylic acids, amides or nitriles of C1-C6 carboxylic acids or their mixtures, preferably water, methanol, dioxane, tetrahydrofuran-THF, 2-methyltetrahydrofuran-2-Me-THF, or their mixture.

6. The process according to claims 1 to 5, **characterized in that** (*R,R*)-4-chlorotartranilate of (*S*)-1-(3-ethoxy-4-methoxyphenyl)-2-(methylsulfonyl)-ethylamine [(*S*)-4aa] or (*R,R*)-di-p-toluoyl-tartrate of (*S*)-1-(3-ethoxy-4-methoxyphenyl)-2-(methylsulfonyl)-ethylamine [(*S*)-4ab] is purified, wherein (*S*)-4aa, or (*S*)-4ab is dissolved in a hot state in distilled water or an alcohol selected from the group of methanol, ethanol, or their mixtures and, after cooling, crystals of the respective substance with the isomeric purity *ee* >99%, measured by HPLC, are separated.

7. The process according to claims 1 to 6, **characterized in that** the salt of formula (*S*)-4a is converted to the free amine of formula (*S*)-2a with the use of a base selected from the group consisting of NaOH, KOH, K₂CO₃ and Na₂CO₃ in the presence of water and a water-immiscible organic solvent selected from the group consisting of C4-C12 ethers, C4-C8 ketones, C3-C12 esters, C1-C7 halogenated hydrocarbons, C5-C12 hydrocarbons and nitrogen derivatives of hydrocarbons, preferably in a mixture of water with dichloromethane, ethyl acetate, ethyl methyl ketone or toluene.

8. The process according to claim 1 to 6, **characterized in that** the reaction with the phthalic anhydride is conducted in the presence of water, an organic solvent, or their mixtures, wherein suitable organic solvents are C2-C5 carboxylic acids, preferably acetic acid, nitriles of C2-C5 carboxylic acids, preferably acetonitrile, and polar aprotic solvents preferably selected from dimethyl formamide, dimethyl acetamide, dimethyl sulfoxide, and further hydrocarbons, preferably toluene, xylenes, α,α,α-trifluorotoluene, chlorobenzene and *n*-butyl chloride.

9. The process according to claims 1 to 6 for preparing (*S*)-{2-[1-(3-ethoxy-4-methoxyphenyl)-2-methylsulfonylethyl]-4-acetylaminoisoindoline-1,3-dione of formula (1), **characterized in that** it comprises a reaction of the racemic amine (2a) with (*R*,*R*)-chlorotartranilic acid ((*S*)-4aa) in the presence of water and hydrochloric acid in the molar ratio of 1:0.65:0.45, and the produced salt is then reacted with 3-acetamidophtalic anhydride in glacial acetic acid under reflux conditions, or the produced (*S*)-1-(3-ethoxy-4-methoxyphenyl)-2-(methylsulfonyl)-ethylammonium-(*R*,*R*)-4-chlorotartranilate is converted in the presence of NaOH to (*S*)-1-(3-ethoxy-4-methoxyphenyl)-2-(methylsulfonyl)-ethylamine, which provides, by a reaction with 3-acetamidophtalic anhydride in glacial acetic acid under reflux conditions, (*S*)-{2-[1-(3-ethoxy-4-methoxyphenyl)-2-methylsulfonylethyl]-4-acetylaminoisoindoline-1,3-dione.

10. (*R*,*R*)-4-chlorotartranilate of (*S*)-1-(3-ethoxy-4-methoxyphenyl)-2-(methylsulfonyl)-ethylamine of formula (*S*)-4aa.

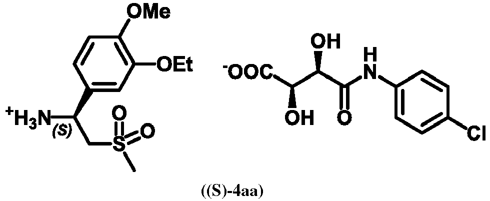

11. (*R*,*R*)-Di-p-toluoyl-tartrate of (*S*)-1-(3-ethoxy-4-methoxyphenyl)-2-(methylsulfonyl)-ethylamine of formula (*S*)-4ab.

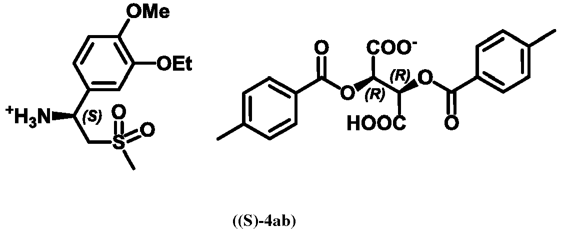

12. Use of (*S*)-1-(3-ethoxy-4-methoxyphenyl)-2-(methylsulfonyl)-ethylamine (*R,R*)*-4-*chlorotartranilate of formula (*S*)-4aa or of (*S*)-1-(3-ethoxy-4-methoxyphenyl)-2-(methylsulfonyl)-ethylamine (*R*,*R*)-di-p-toluoyl-tartrate of formula (*S*)-4ab, as defined in claims 10 and 11, in the synthesis of (*S*)-{2-[1-(3-ethoxy-4-methoxyphenyl)-2-methylsulfonylethyl] -4-acetylaminoisoindoline-1,3-dione.

## Patentansprüche

1. Verfahren zur Herstellung von (*S*)-{2-[1-(3-Ethoxy-4-methoxyphenyl)-2-methylsulfonylethyl]-4-acetylaminoisoindolin-1,3-dion der Formel 1 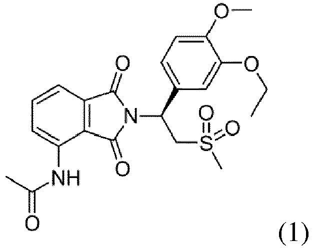 **dadurch gekennzeichnet, dass** es eine Reaktion des racemischen Amins der Formel 2a 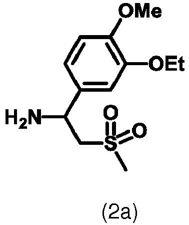 mit (*R,R*)-4-Chlortartranilsäure oder (*R,*R)-Di-*p*-toluoylweinsäure in einem geeigneten Lösungsmittel, vorzugsweise unter Verwendung eines Additivs, wobei das Salz der Formel (*S*)-4a 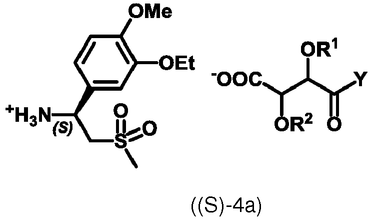 nämlich (*S*)-1-(3-Ethoxy-4-methoxyphenyl)-2-(methylsulfonyl)ethylamin (*R,R*)-4-Chlortartranilat beziehungsweise (*S*)-1-(3-Ethoxy-4-methoxyphenyl)-2-(methylsulfonyl)ethylamin (*R*,*R*)-Di-p-toluoyltartrat, erhalten wird,
und eine anschließende Umsetzung des erhaltenen Salzes der Formel (*S*)-4a mit 3-Acetamidophthalsäureanhydrid der Formel 5, 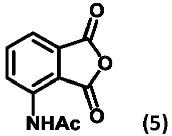 wobei (*S*)-12-[1-(3-Ethoxy-4-methoxyphenyl)-2-methylsulfonylethyl]-4-acetylaminoisoindolin-1,3-dion der Formel 1 erhalten wird,
oder Umwandlung des hergestellten Salzes der Formel (*S*)-4a in einem geeigneten Lösungsmittel unter Verwendung einer Base in das freie Amin der Formel (*S*)-2a, 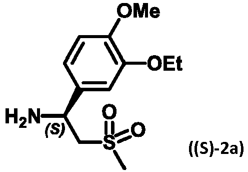 wobei durch eine anschließende Umsetzung mit 3-Acetamidophthalsäureanhydrid der Formel 5 (*S*)-12-[1-(3-Ethoxy-4-methoxyphenyl)-2-methyl-sulfonylethyl]-4-acetylaminoisoindolin-1,3-dion der Formel 1 erhalten wird, umfasst.

2. Verfahren nach Anspruch 1, **dadurch gekennzeichnet, dass** das Molverhältnis des racemischen Amins und des Derivats der Weinsäure 1:0,5 bis 1:0,65 beträgt.

3. Verfahren nach den Ansprüchen 1 bis 2, **dadurch gekennzeichnet, dass** das Additiv ausgewählt ist aus der Gruppe bestehend aus Mineralsäuren, umfassend Schwefel-, Phosphor-, Perchlor-, Salz- und Bromwasserstoffsäure, und organischen Säuren, umfassend Ameisensäure oder Sulfonsäuren, vorzugsweise Salzsäure.

4. Verfahren nach Anspruch 3, **dadurch gekennzeichnet, dass** das Molverhältnis von racemischem Amin, Weinsäurederivat und Additiv 1:0,50 bis 0,65 :0,45 bis 0,60, vorzugsweise 1:0,65:0,45 beträgt.

5. Verfahren nach einem der vorhergehenden Ansprüche, **dadurch gekennzeichnet, dass** das Lösungsmittel für die Umsetzung mit den Derivaten der Weinsäure aus der Gruppe bestehend aus Wasser, aliphatischen C1-C6-Alkoholen, C4-C12-Ethern, C3-C8-Ketonen, C3-C12-Estern, halogenierten C1-C7-Kohlenwasserstoffen, C5-C12-Kohlenwasserstoffen, Stickstoffderivaten von Kohlenwasserstoffen und Carbonsäuren, Amiden oder Nitrilen von C1-C6-Carbonsäuren oder deren Gemische, vorzugsweise Wasser, Methanol, Dioxan, Tetrahydrofuran-THF, 2-Methyltetrahydrofuran-2-Me-THF oder deren Gemische, ausgewählt ist.

6. Verfahren nach Ansprüchen 1 bis 5, **dadurch gekennzeichnet, dass** (*R,R*)-4-Chlortartranilat von (*S*)-1-(3-Ethoxy-4-methoxyphenyl)-2-(methylsulfonyl)ethylamin[(*S*)-4aa] oder (*R*,*R*)-Di-p-toluoyltartrat von (*S*)-1-(3-Ethoxy-4-methoxyphenyl)-2-(methylsulfonyl)ethylamin [(*S*)-4ab] gereinigt wird, wobei (*S*)-4aa oder (S)-4ab in heißem Zustand in destilliertem Wasser oder einem Alkohol, ausgewählt aus der Gruppe Methanol, Ethanol oder deren Gemische, gelöst wird und nach dem Abkühlen Kristalle der jeweiligen Substanz mit der durch HPLC gemessenen Isomerenreinheit *ee* >99 % abgetrennt werden.

7. Verfahren nach Ansprüchen 1 bis 6, **dadurch gekennzeichnet, dass** das Salz der Formel (*S*)-4a unter Verwendung einer Base, ausgewählt aus der Gruppe bestehend aus NaOH, KOH, K₂CO₃ und Na₂CO₃ in Gegenwart von Wasser und einem mit Wasser nicht mischbaren organischen Lösungsmittel, ausgewählt aus der Gruppe bestehend aus C4-C12-Ethern, C4-C8-Ketonen, C3-C12-Estern, halogenierten C1-C7-Kohlenwasserstoffen, C5-C12-Kohlenwasserstoffen und Stickstoffderivaten von Kohlenwasserstoffen, vorzugsweise in einem Gemisch von Wasser mit Dichlormethan, Ethylacetat, Ethylmethylketon oder Toluol, in das freie Amin der Formel (*S*)-2a überführt wird.

8. Verfahren nach Anspruch 1 bis 6, **dadurch gekennzeichnet, dass** die Umsetzung mit dem Phthalsäureanhydrid in Gegenwart von Wasser, einem organischen Lösungsmittel oder Gemischen davon durchgeführt wird, wobei geeignete organische Lösungsmittel C2-C5-Carbonsäuren, vorzugsweise Essigsäure, Nitrile von C2-C5-Carbonsäuren, vorzugsweise Acetonitril, und aprotisch-polare Lösungsmittel, vorzugsweise ausgewählt aus Dimethylformamid, Dimethylacetamid, Dimethylsulfoxid, und weitere Kohlenwasserstoffe, vorzugsweise Toluol, Xylole, α,α,α-Trifluortoluol, Chlorbenzol und *n*-Butylchlorid, sind.

9. Verfahren nach den Ansprüchen 1 bis 6 zur Herstellung von (*S*)-{2-[1-(3-Ethoxy-4-methoxyphenyl)-2-methylsulfonylethyl]-4-acetylaminoisoindolin-1,3-dion der Formel (1), **dadurch gekennzeichnet, dass** es eine Umsetzung des racemischen Amins (2a) mit (*R*,*R*)-Chlortartranilsäure ((*S*)-4aa) in Gegenwart von Wasser und Salzsäure im Molverhältnis von 1:0,65:0,45 umfasst, und das erhaltene Salz dann mit 3-Acetamidophthalsäureanhydrid in Eisessig unter Rückflußbedingungen umgesetzt oder das erhaltene (*S*)-1-(3-Ethoxy-4-methoxyphenyl)-2-(methylsulfonyl)ethylammonium-(*R*,*R*)-4-chlortartranilat in Gegenwart von NaOH zu (*S*)-1-(3-Ethoxy-4-methoxyphenyl)-2-(methylsulfonyl)ethylamin umgewandelt wird, wobei durch Umsetzung mit 3-Acetamidophthalsäureanhydrid in Eisessig unter Rückflussbedingungen (*S*)-{2-[1-(3-Ethoxy-4-methoxyphenyl)-2-methylsulfonylethyl]-4-acetylaminoisoindolin-1,3-dion erhalten wird.

10. (*R,R*)-4-Chlortartranilat von (*S*)-1-(3-Ethoxy-4-methoxyphenyl)-2-(methylsulfonyl)ethylamin der Formel (*S*)-4aa.

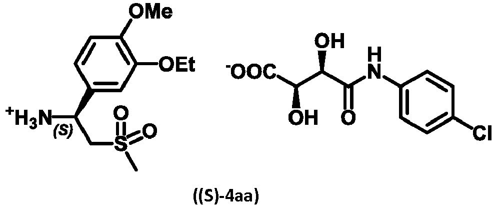

11. (*R*,*R*)-Di-p-toluoyltartrat von (*S*)-1-(3-Ethoxy-4-methoxyphenyl)-2-(methylsulfonyl)ethylamin der Formel (*S*)-4ab.

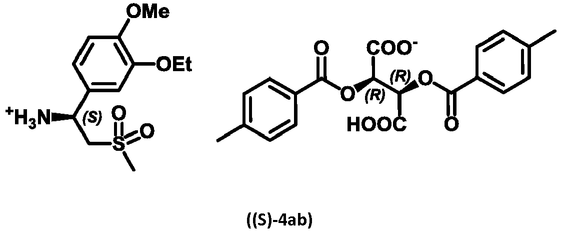

12. Verwendung von (*S*)-1-(3-Ethoxy-4-methoxyphenyl)-2-(methylsulfonyl)-ethylamin (*R,R*)-4-Chlortartranilat der Formel (*S*)-4aa oder von (*S*)-1-(3-Ethoxy-4-methoxyphenyl)-2-(methylsulfonyl)ethylamin (*R,R*)-Di-p-toluoyltartrat der Formel (*S*)-4ab, wie in den Ansprüchen 10 und 11 definiert, in der Synthese von (*S*)-{2-[1-(3-Ethoxy-4-methoxyphenyl)-2-methylsulfonylethyl]-4-acetylaminoisoindolin-1,3-dion.

## Revendications

1. Un procédé de préparation de (S)-{2-[1-(3-éthoxy-4-méthoxyphényl)-2-méthylsulfonyléthyl]-4-acétylaminoisoindoline-1,3-dione de formule 1, 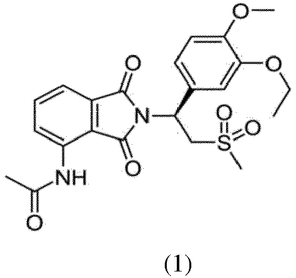 **caractérisé en ce qu'**il comprend une réaction de l'amine racémique de formule 2a 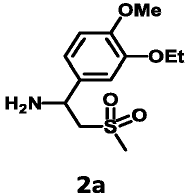 avec l'acide (R,R)-4-chlorotartranilique ou l'acide (R,R)-di-p-toluoyl-tartrique dans un solvant approprié, de préférence avec utilisation d'un additif, pour produire le sel de formule (S)-4a, 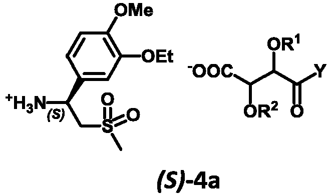 à savoir le (S)-1-(3-éthoxy-4-méthoxyphényl)-2-(méthylsulfonyl)-éthylamine(R,R)-4-chlorotartranilate et le (S)-1-(3-éthoxy-4-méthoxyphényl)-2-(méthylsulfonyl)-éthylamine (R,R)-di-p-toluoyl-tartrate, respectivement,
et la réaction ultérieure du sel obtenu de formule (S)-4a avec l'anhydride 3-acétamidophtalique de formule 5 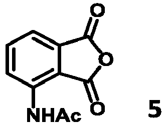 pour produire la (S)-{2-[1-(3-éthoxy-4-méthoxyphényl)-2-méthylsulfonyléthyl]-4-acétylaminoisoindoline-1,3-dione de formule 1,
ou la conversion du sel produit de formule (S)-4a dans un solvant approprié, avec utilisation d'une base de l'amine libre de formule (S)-2a, 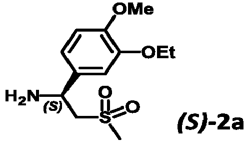 qui, par une réaction ultérieure avec l'anhydride acétamidophtalique de formule 5 conduit à l'obtention de la (S)-{2-[1-(3-éthoxy-4-méthoxyphényl)-2-méthylsulfonyléthyl]-4-acétylaminoisoindoline-1,3-dione de formule 1.

2. Le procédé selon la revendication 1, **caractérisée en ce que** le rapport molaire amine racémique/dérivé de l'acide tartrique est compris entre 1/0,5 et 1/0,65.

3. Le procédé selon les revendications 1 à 2, **caractérisé en ce que** ledit additif est choisi dans le groupe constitué par les acides minéraux comprenant les acides sulfurique, phosphorique, perchlorique, chlorhydrique et bromhydrique, et les acides organiques comprenant l'acide formique ou les acides sulfoniques, de préférence l'acide chlorhydrique.

4. Le procédé selon la revendication 3, **caractérisé en ce que** le rapport molaire amine racémique/dérivé de l'acide tartrique/additif est de 1/0,50 à 0,65/0,45 à 0,60, de préférence de 1/0,65/0,45.

5. Le procédé selon l'une quelconque des revendications précédentes, **caractérisé en ce que** le solvant pour la réaction avec les dérivés de l'acide tartrique est choisi dans le groupe constitué par l'eau, les alcools aliphatiques en C1-C6, les éthers en C4-C12, les cétones en C3-C8, les esters en C3-C12, les hydrocarbures halogénés en C1-C7, les hydrocarbures en C5-C12, les dérivés azotés d'hydrocarbures et d'acides carboxyliques, les amides ou les nitriles d'acides carboxyliques en C1-C6 ou leurs mélanges, de préférence l'eau, le méthanol, le dioxane, le tétrahydrofurane-THF, le 2-méthyltétrahydrofuranne-2-Me-THF, ou leur mélange.

6. Le procédé selon les revendications 1 à 5, **caractérisé en ce que** le (R,R) - 4-chlorotartranilate de (S)-1-(3-éthoxy-4-méthoxyphényl)-2-(méthylsulfonyl)-éthylamine[(S)-4aa] ou (R,R)-di-p-toluoyl-tartrate de (S)-1-(3-éthoxy-4-méthoxyphényl)-2-(méthylsulfonyl)-éthylamine[(S)-4ab] est purifié, dans lequel procédé le (S)-4aa ou le (S)-4ab est dissous à chaud dans de l'eau distillée ou un alcool choisi dans le groupe du méthanol, de l'éthanol ou de leurs mélanges et, après refroidissement, sont séparés des cristaux de la substance respective présentant la pureté isomérique, mesurée par HPLC, ee> 99%.

7. Le procédé selon les revendications 1 à 6, **caractérisé en ce que** le sel de formule (S)-4a est converti en l'amine libre de formule (S)-2a en utilisant une base choisie dans le groupe constitué de NaOH, KOH, K₂CO₃ et Na₂CO₃ en présence d'eau et d'un solvant organique non miscible à l'eau choisi dans le groupe constitué par les éthers en C4 à C12, les cétones en C4 à C8, les esters en C3 à C12, les hydrocarbures halogénés en C1 à C7, les hydrocarbures en C5 à C12 et les dérivés azotés d'hydrocarbures, de préférence en utilisant un mélange d'eau et de dichlorométhane, acétate d'éthyle, éthylméthylcétone ou toluène.

8. Le procédé selon l'une des revendications 1 à 6, **caractérisé en ce que** la réaction avec l'anhydride phtalique est effectuée en présence d'eau, d'un solvant organique ou de leurs mélanges, les solvants organiques appropriés étant des acides carboxyliques en C2-C5, de préférence l'acide acétique, des nitriles d'acides carboxyliques en C2-C5, de préférence l'acétonitrile, et des solvants aprotiques polaires choisis de préférence parmi le diméthylformamide, le diméthylacétamide, le diméthylsulfoxyde et d'autres hydrocarbures, de préférence le toluène, les xylènes, les α,α,α-trifluorotoluènes, le chlorobenzène et le chlorure de n-butyle.

9. Le procédé selon les revendications 1 à 6 pour la préparation de (S)-{2-[1-(3-éthoxy-4-méthoxyphényl)-2-méthylsulfonyléthyl]-4-acétylaminoisoindoline-1,3-dione de formule (1), **caractérisé en ce qu'**il comprend une réaction de l'amine racémique (2a) avec l'acide (R,R)-chlorotartranilique ((S)-4aa) en présence d'eau et d'acide chlorhydrique dans un rapport molaire de 1/0,65/0,45 et le sel produit est ensuite mis à réagir avec l'anhydride 3-acétamidophtalique dans de l'acide acétique glacial dans des conditions de reflux, ou le (S)-1-(3-éthoxy-4-méthoxyphényl)-2-(méthylsulfonyl)-éthylammonium produit (R,R)-4-chlorotartranilate est converti en présence de NaOH en (S)-1-(3-éthoxy-4-méthoxyphényl)-2-(méthylsulfonyl)-éthylamine, ce qui fournit, par réaction avec l'anhydride 3-acétamidophtalique dans l'acide acétique glacial dans des conditions de reflux, la (S)-{2-[1-(3-éthoxy-4-méthoxyphényl)-2-méthylsulfonyléthyl]-4-acétylaminoisoindoline-1,3-dione.

10. Le (R,R)-4-chlorotartranilate de (S)-1-(3-éthoxy-4-méthoxyphényl)-2-(méthylsulfonyl)-éthylamine de formule (S)-4aa.

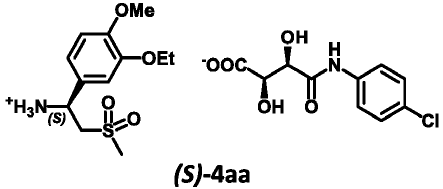

11. Le (R,R)-di-p-toluoyl-tartrate de (S)-1-(3-éthoxy-4-méthoxyphényl)-2-(méthylsulfonyl)-éthylamine de formule (S)-4ab.

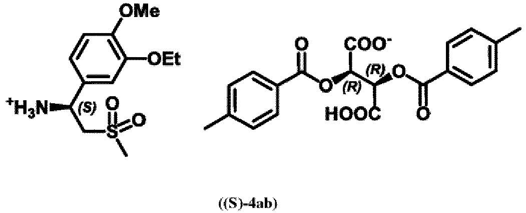

12. Utilisation de (S)-1-(3-éthoxy-4-méthoxyphényl)-2-(méthylsulfonyl)-éthylamine (R,R)-4-chlorotartranilate de formule (S)-4aa ou de (S)-1-(3-éthoxy-4-méthoxyphényl)-2-(méthylsulfonyl)-éthylamine (R,R)-di-p-toluoyl-tartrate de formule (S)-4ab, comme défini dans les revendications 10 et 11, dans la synthèse de (S)-{2-[1-(3-éthoxy-4-méthoxyphényl)-2-méthylsulfonyléthyl]-4-acétylaminoisoindo-line-1,3-dione.
